(19) Europäisches Patentamt
European Patent Office
Office européen des brevets

(11) Numéro de publication: **0 112 252**
**B1**

(12) **FASCICULE DE BREVET EUROPÉEN**

(45) Date de publication du fascicule du brevet: **07.02.90**

(51) Int. Cl.[5]: **C 07 D 455/03,** A 61 K 31/47

(21) Numéro de dépôt: **83402418.4**

(22) Date de dépôt: **14.12.83**

(54) **Enantiomères lévogyres des dérivés de la tétrahydro-5,6,13,13a 8H-dibenzo(a, g)quinolizine, procédés d'obtention, compositions pharmaceutiques les contenant et application.**

(30) Priorité: **14.12.82 FR 8220975**

(43) Date de publication de la demande:
**27.06.84 Bulletin 84/26**

(45) Mention de la délivrance du brevet:
**07.02.90 Bulletin 90/06**

(84) Etats contractants désignés:
**AT BE CH DE GB IT LI LU NL SE**

(56) Documents cité:
**EP-A-0 028 959**
**GB-A-1 004 077**

**CHEMICAL ABSTRACTS, vol. 82, no. 3, 1975, page 460, no. 43626u, Columbus, Ohio, US S.TEITEL et al.: "Tetrahydroprotoberberines derived from R- and S-1-(3,4 - dihydroxybenzyl) - 1,2,3,4 - tetrahydroisoquinoline"**

**BERICHTE DER DEUTSCHEN CHEMISCHEN GESELLSCHAFT, vol. 63, no. 358, 1930, pages 2343-2347, Weinheim, DE W. LEITHE: "UEber das optische Drehungsvermögen und die Konfiguration einiger Basen vom Typus des Tetrahydro-berberins"**

**Le dossier contient des informations techniques présentées postérieurement au dépôt de la demande et ne figurant pas dans le présent fascicule.**

(73) Titulaire: **U.R.P.H.A.**
**34, Boulevard de Clichy**
**F-75018 Paris (FR)**

(72) Inventeur: **Stambach, Jean-Franfois**
**10 Rue d'Andlau**
**67000 Strasbourg (FR)**
Inventeur: **Jung, Louis**
**205 Route d'Oberhaustergen**
**67200 Strasbourg (FR)**
Inventeur: **Schott, Claire**
**7 Square du Château**
**67300 Schiltigheim (FR)**
Inventeur: **Heitz, Christiane**
**8 Rue de Zinsel**
**67300 Schiltigheim (FR)**
Inventeur: **Stoclet, Jean-Claude**
**13 Boulevard Sébastien Bach**
**67000 Strasbourg (FR)**
Inventeur: **Schutz, Fabienne**
**2 Rue du Faisan**
**67120 Molsheim (FR)**

(74) Mandataire: **Phélip, Bruno**
**c/o Cabinet Harlé & Phélip 21, rue de La Rochefoucauld**
**F-75009 Paris (FR)**

Il est rappelé que: Dans un délai de neuf mois à compter de la date de publication de la mention de la délivrance du brevet européen toute personne peut faire opposition au brevet européen délivré, auprès dé l'Office européen des brevets. L'opposition doit être formée par écrit et motivée. Elle n'est réputée formée qu'après paiement de la taxe dipposition (Art. 99(1) Convention sur le brevet européen).

EP 0 112 252 B1

LIBERGRAF, STOCKHOLM 1990

**Description**

La présente invention concerne les énantiomères lévogyres des dérivés de la tétrahydro-5,6,13, 13a 8H-dibenzo [a,g] quinolizine. Elle concerne également l'obtention desdits énantiomères. L'invention a encore pour objet l'application thérapeutique de ces nouveaux composés et les compositions pharmaceutiques les contenant.

La tétrahydro-5,6,13,13a 8H dibenzo [a,g] quinolizine, dont la dénomination commune française est "Berbine" répond à la formule I ci-après:

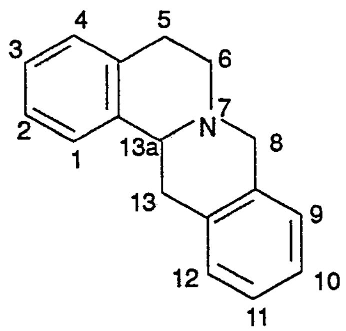

I

La "Berbine" inverse les effets hypertenseurs de l'adrénaline chez le chien [Raymond Hamet, Bulletin de l'Académie Nationale de Médecine 1952, 22 et 23, 408; Raymond Hamet C.R. Acad. Sci. 1953, 236, 1916]. La 1-berbine possède chez le chat comme chez le chien une forte activité sympathicolytique [Raymond Hamet, C.R. Acad. Sci. Paris, t 259 1964, p. 4397-4399]. La Berbine exerce une action hypotensive chez des patients hypertendus [Faquet, Lisles et Combaz, Gazette Medicale de France, 1954, 61, 1615]. La séparation des énantiomères de la berbine est décrite par Wolfgang Leithe, Berichte 63 2343 (1930) par recristallisations successives de certains de ses sels avec des acides optiquement actifs, selon une méthode classique.

Le brevet EU-0 028 959 décrit l'obtention du mélange racémique de certains dérivés de la tétrahydro-5,6,13,13a 8H dibenzo [a,g] quinolizine, ainsi que leur utilisation pour le traitement des troubles cardiovasculaires, mais la séparation des énantiomères, et/ou leurs éventuelles propriétés thérapeutiques remarquables n'y est pas suggéré.

Dans la publication Hétérocycles (1974) 2(5) 625-30 la préparation de certains dérivés de la Berbine est décrite à partir de chacun des énantiomères, séparés, des isoquinoléines correspondantes. Le procédé d'obtention de chaque énantiomère est tout-à-fait différent de celui selon l'invention puisqu'on réalise une synthèse stéréospécifique, à partir de chaque isomère de l'isoquinoléine convenablement substituée. Les essais décrits prouvent que les composes ainsi obtenus, qui répondent à la formule Ib ci-dessous n'ont pas d'activité dopaminergique.

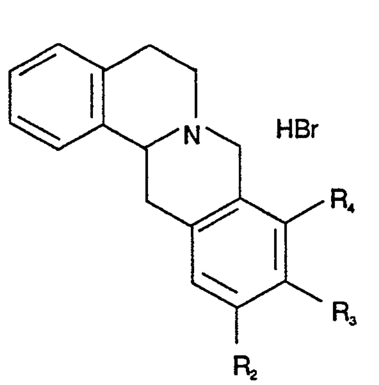

Ib

dans laquelle si $R_4$ est H, $R_2$ et $R_3$ représentent OH ou $OCH_3$ et si $R_2$ est H, $R_3$ et $R_4$ représentent OH.

Enfin, le brevet GB-1 004 077 concerne des compositions contenant des dibenzoquinolizines qui possèdent une activité tranquillisante, antidépressive et antiémétique. Il est indiqué que ces dibenzoquinolizines peuvent être sous la forme du racémique ou sous forme des énantiomères d ou 1, mais l'isolement de ces derniers n'est pas décrit et leurs propriétés respectives restent inconnues.

Ces dibenzoquinolizines répondent à la formule générale ci-après:

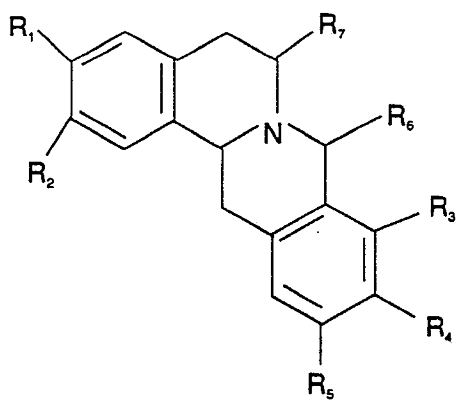

Ia

dans laquelle

$R_1$ et $R_2$ représentent notamment l'hydrogène, les groupes hydroxy, méthoxy ou éthoxy;
$R_3$ et $R_5$ représentent l'hydrogène ou le groupe méthoxy;
$R_4$ représente l'hydrogène, les groupes hydroxy, méthoxy ou éthoxy et
R6 et R7 représentent l'hydrogène ou le groupe méthyle.

Il faut noter que par le procédé de synthèse décrit dans ce brevet GB-1 004 077, on ne peut obtenir les dérivés hydroxylés en position 3, c'est-à-dire les composés de formule Ia ci-dessus dans laquelle $R_1$ est le groupe hydroxy.

En effet, les conditions opératoires du procédé mentionné à la page 2, lignes 59 à 64 de ce brevet GB-1 004 077 se révèlent être trop dures (acidité et chaleur) pour que le groupe benzyloxy (protecteur de la fonction hydroxy) ne soit pas décomposé au cours de la réaction de cyclisation des phénylacétamides N-substitués en 3,4-dihydro-isoquinoléines, réalisée en présence d'oxychlorure de phosphore et par chauffage.

Dans la demande de brevet FR-A-2 469 413 au nom de la demanderesse on a décrit des dérivés de la "Berbine" qui présentent une activite pharmacologique intéressante, notamment dans le domaine cardio-vasculaire pour lutter contre les troubles du rythme et les insuffisances cardiaques et coronariennes.

Les dérivés de la "Berbine" décrits dans cette demande de brevet FR-A-2 469 413 répondent à la formule générale II:

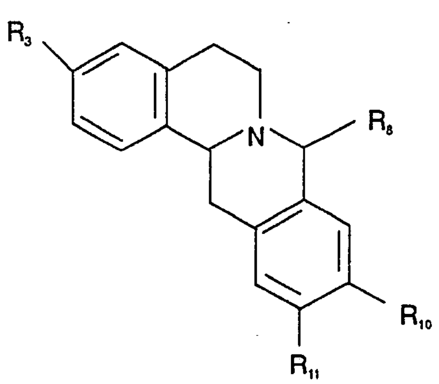

II

dans laquelle:

$R_3$ est un radical -OR ou -SR, R étant l'hydrogène, un groupe alkyle ou ayrle ou un groupe de formule R' – C –, R' étant un groupe alkyle ou aryle:
||
O

H
/
$R_8$ est = O ou
\
H

$R_{10}$ et $R_{11}$, identiques ou différents, représentent l'hydrogène ou un groupe alcoxy inférieur ou aryloxy à la condition que, lorsque

H
/
$R_8$ est
\
H

$R_{10}$ et $R_{11}$ sont l'hydrogène ou un groupe alcoxy inférieur, $R_3$ est différent du groupe OR dans lequel R est un groupe alkyle.

On a maintenant trouvé que les énantiomères lévogyres de dérivés de la Berbine ont une importante activité hypotensive, ils inhibent compétitivement l'effet vasoconstricteur de la phényléphrine, c'est-à-dire qu'ils ont une activité α-bloquante alors que les racémiques correspondants se comportent comme des antagonistes non compétitifs de la phényléphrine et par conséquent peuvent en thérapeutique avoir des effets secondaires indésirables. Ces activités sont toutes les deux stéréospécifiques. A la différence des dibenzo-quinolizines qui possèdent une activité tranquillisante, antidépressive et antiémétique (voir le brevet GB-1 004 077), les composés selon l'invention conviennent dans des applications thérapeutiques pour traiter les maladies cardiovasculaires notamment l'hypertension artérielle, l'insuffisance coronarienne et l'insuffisance cardiaque.

La présente invention concerne donc les énantiomères lévogyres de dérivés de la tétrahydro-5, 6,13, 13a 8H-dibenzo [a,g] quinolizine (berbine) et leurs sels pharmaceutiquement acceptables. Les composés de l'invention répondent à la formule générale III

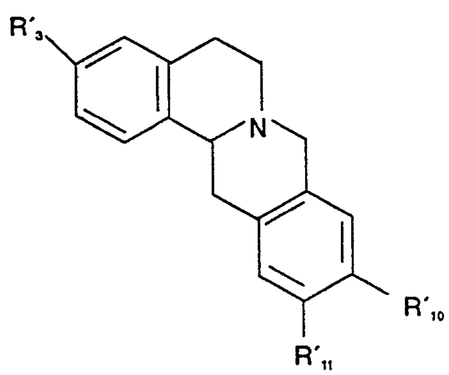

III

dans laquelle:

- $R'_3$ est l'hydrogène, un groupe alkyle ou aryle ou un radical -OR ou -SR, R étant l'hydrogène, un groupe alkyle ou aryle ou un groupe de formule:
  R"–C–, R" étant un groupe alkyle ou aryle
  ||
  O
- $R'_{10}$ et $R'_{11}$, identiques ou différents, représentent l'hydrogène, un groupe hydroxy, alcoxy, ou aryloxy ou $R'_{10}$ et $R'_{11}$ forment ensemble un groupe -O-$(CH_2)$n-O- dans lequel <u>n</u> vaut 1 à 3, à la condition que $R'_3$, $R'_{10}$ et $R'_{11}$ ne

soient pas simultanément l'hydrogène; que, si $R'_{10}$ et $R'_{11}$ sont l'hydrogène, $R'_3$ ne soit pas un groupe alcoxy; et que, lorsque $R'_3$ est l'hydrogène, $R'_{10}$ et $R'_{11}$ ne soient pas un groupe hydroxy ou méthoxy;

les groupes alkyle et alcoxy ayant 1 à 12 atomes de carbone, et les groupes aryle étant choisis parmi les radicaux phényle ou phénylalkyle, où le groupe alkyle renferme de 1 à 4 atomes de carbone, et les hétérocycles aromatiques ayant 4 à 7 atomes de carbone et de 1 à 4 hétéroatomes pouvant être l'oxygène, l'azote ou le soufre.

Dans la présente description, le terme "alkyle" désigne des groupes hydrocarbonés aliphatiques contenant 1 à 12 atomes de carbone, à chaîne droite ou ramifiée. On préfère les groupes alkyle inférieur, c'est-à-dire les groupes alkyle contenant 1 à 4 atomes de carbone.

Le terme "aryle" désigne les groupes aromatiques non hétérocycliques du type phényle, benzyle et les homologues supérieurs, substitués ou non, ainsi que les groupes aromatiques hétérocycliques ayant 4 à 7 atomes de carbone dans le cycle aromatique, et 1 à 4 hétéroatomes pouvant être l'oxygène, l'azote, le soufre, du type furane, pyridine, oxazole, ainsi que leurs dérivés saturés respectivement cyclohexane et tétrahydrofurane, pypéridine, oxazolidine.

L'expression "alcoxy inférieur" désigne les groupes de formule $R'_a$-O dans laquelle $R'_a$ est un groupe alkyle inférieur, tel que défini ci-dessus.

Les composés selon l'invention sont obtenus à partir des racémiques correspondants par des procédés classiques de séparation utilisant des recristallisations successives d'un sel formé entre les dérivés de la berbine sous forme de bases et un réactif acide de dédoublement dans un solvant approprié.

Les racémiques des dérivés de la berbine selon l'invention sont obtenus par exemple par le procédé décrit dans la demande de brevet FR-A-2 469 413.

Sous son aspect général, ce procédé de synthèse consiste à:

1) condenser, avec du nitrométhane, le benzaldéhyde substitué en position 3 par un groupe $R'_3$ protégé, pour former le nitrostyrène correspondant;
2) réduire le nitrostyrène obtenu pour former la phénéthylamine correspondante;
3) condenser la phénéthylamine obtenue avec l'acide phénylacétique substitué de formule:

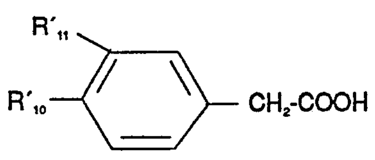

pour former le phénacétamide correspondant;
4) procéder à une première cyclisation pour obtenir la 3,4-dihydroisoquinoléine correspondante;
5) réduire la dihydroisoquinoléine en tétrahydroisoquinoléine;
6) fixer, par condensation avec de l'acide formique ou du phosgène, un groupe formyle ou chloroformyle sur l'atome d'azote de l'isoquinoléine;
7) procéder à une seconde cyclisation;
8) réduire le produit résultant pour former le dérivé de la berbine désiré;
9) éventuellement, éliminer le groupement protecteur en position 3; et
10) éventuellement, estérifier ou éthérifier le produit résultant.

Comme cela est indiqué ci-dessus, le procédé de synthèse des composés de l'invention comporte deux variantes au niveau de l'étape 6; l'une des variantes consiste en la formylation de la tétrahydroisoquinoléine obtenue à l'étape 5 et l'autre consiste en la chloroformylation de ladite tétrahydroisoquinoléine qui permet l'obtention des composés de formule II dans laquelle $R_8$ est l'oxygène, des radicaux $R'_3$, $R'_{10}$ et $R'_{11}$ étant tels que définis précédemment.

Pour plus de détails sur ce procédé de synthèse et les variantes de mise en oeuvre, on pourra se référer à la demande de brevet FR-A-2 469 413 citée dans la présente demande à titre de référence.

Le procédé selon la présente invention pour l'obtention des isomères optiques purs consiste à faire réagir la fonction amine du racémique qui est une base avec un acide de dédoublement approprié pour former les sels des 2 énantiomères et à séparer le sel de l'énantiomère recherché par des recristallisations successives dans un solvant approprié.

Avantageusement, on utilise comme acide de dédoublement:
l'acide (-) diparatoluoyl-L-tartrique,
l'acide (+) diparatoluoyl-D-tartrique,
qui selon les cas permettront l'isolement de l'un ou l'autre des énantiomères.

Les solvants de recristallisation utilisés selon l'invention sont par exemple le méthanol, l'éthanol, l'acétate

d'éthyle, le chloroforme et composés similaires qui conviennent pour la recristallisation du composé considéré.

Les isomères optiques purs de formule III ci-dessus dans laquelle $R'_3$ est un radical hydroxy peuvent être obtenus par hydrolyse des isomères optiques purs des composés correspondants de formule III dans laquelle $R'_3$ est le groupe de formule R"-COO-. L'hydrolyse est avantageusement réalisée en milieu acide, par exemple en milieu acétochlorhydrique.

Ces isomères optiques purs des composés de formule III dans laquelle $R'_3$ est le groupe hydroxy peuvent ensuite être estérifiés pour donner d'autres stéréoisomères purs de composés selon l'invention. Une telle estérification peut être réalisée par réaction dudit composé de formule III avec un chlorure d'acide de formule

$$R''-\underset{\underset{O}{\|}}{C}-Cl$$

en présence d'une base, telle que la triéthylamine dans un solvant approprié, tel que le chloroforme. Après isolement de l'ester formé, celui-ci est recristallisé dans un solvant approprié; le produit ainsi obtenu répond à la formule III dans laquelle R'3 est un radical $R''-\underset{\underset{O}{\|}}{C}-O-$.

Toutes les étapes ci-dessus sont réalisées à la température ambiante ou à toute autre température appropriée pour ces types de réactions et dont la détermination est à la portée de l'homme de l'art par des essais de routine.

Les composés selon l'invention possèdent des propriétés pharmacologiques intéressantes, notamment dans le domaine cardio-vasculaire pour lutter à la fois contre les troubles du rythme, les insuffisances cardiaques et coronariennes et l'hypertension artérielle. Les composés de l'invention, comme on l'a indiqué précédemment, inhibent compétitivement l'effet vasoconstricteur de la phényléphrine, qui est le composé suivant: (R)-3-hydroxy-α-[(méthylamino)méthyl]benzèneméthanol, ont une activité α-bloquante, pour certains spécifiques des récepteurs $\alpha_1$, et un effet hypotenseur.

La présente invention concerne donc également les compositions pharmaceutiques contenant, à titre d'ingrédient actif, l'énantiomère lévogyre d'un composé répondant à la formule III dans laquelle:

- $R'_3$ est hydrogène, un groupe alkyle, aryle, un radical OR ou SR, où R est l'hydrogène, un groupe alkyle, aryle ou R"–C–, avec R" représentant un groupe alkyle ou aryle,
- $R'_{10}$ et $R'_{11}$, identiques ou différents, représentent l'hydrogène, un groupe hydroxy, alcoxy, aryloxy ou forment ensemble un groupe $-O-(CH_2)_nO-$ dans lequel n vaut 1 à 3,
  à la condition que $R'_3$, $R'_{10}$ et $R'_{11}$ ne soient simultanément l'hydrogène; et à la condition que si $R'_{10}$ et $R'_{11}$ sont l'hydrogène; où un groupe alcoxy inférieur, $R'_3$ ne soient pas un groupe alcoxy;

les groupes alkyle et alcoxy, ainsi que les groupes aryle étant tels que difinis ci-dessus, en combinaison avec un excipient approprié. Les compositions pharmaceutiques selon l'invention peuvent se présenter notamment sous des formes convenant à l'administration par voie orale ou parentérale.

Pour une administration par voie orale, on pourra utiliser les compositions de l'invention sous la forme de comprimés, de gélules, de sirops, de gouttes, les excipients utilisés étant des excipients classiques pour la formation de ces formes pharmaceutiques, bien évidemment inertes vis-à-vis de l'ingrédient actif. La dose thérapeutique dépend du trouble à traiter mais on administrera, en général de 50 à 500 mg par jour des énantiomères lévogyres selon l'invention.

L'invention va maintenant être décrite plus en détail dans les exemples ci-après donnés à titre non limitatif. Dans ces exemples, on a utilisé comme composés de départ pour l'obtention des isomères selon l'invention, les racémiques préparés selon le procédé décrit dans le brevet FR-A-2 469 413. Ainsi, on a utilisé comme produits de départ les composés ci-après obtenus par les modes opératoires décrits dans les exemples de la demande de brevet FR-A-2 469 413 indiqués pour chaque composé.

| **Racémique**<br>Composé | Dénomination | $R'_3$ | $R'_{10}$ | $R'_{11}$ | Exemple de la demande préparé selon FR-A-2 469 413 |
|---|---|---|---|---|---|
| hydroxy-3 tétrahydro-5,6,13,13a 8H-dibenzo [a,g] quinolizine ou hydroxy-3 berbine | STB 4 | HO | H | H | Exemple 5 |
| diméthoxy-10,11 hydroxy-3 tétrahydro-5,6,13,13a 8H-dibenzo [a,g] quinolizine ou diméthoxy-10,11 hydroxy-3 berbine | STB 6 | HO | $CH_3O$ | $CH_3O$ | Exemple 1 |
| diméthoxy-10,11 phénacétyloxy-3 tétrahydro 5,6,13,13a 8H-dibenzo [a,g] quinolizine ou diméthoxy-10,11 phénacétyloxy-3-berbine | STB 52 | $C_6H_5CH_2CO_2$ | $CH_3O$ | $CH_3O$ | Exemple 6 |

| | | | | | |
|---|---|---|---|---|---|
| phénacétyloxy-3 tétrahydro-5,6,13,13a 8H-dibenzo [a,g] quinolizine ou phénacétyloxy-3 berbine | STB 69 | $C_6H_5CH_2CO_2$ | H | H | Exemple 6 |

A des fins de comparaison, on a également préparé les isomères dextrogyres correspondants.

**Exemple 1:**

**Préparation des isomères optiques purs de la diméthoxy-10,11 hydroxy-3 tétrahydro-5,6,13,13a 8 H-dibenzo [a,g] quinolizine ou diméthoxy-10,11 hydroxy-3 berbine (STB 6).**

**Isomère (-) STB 6**

La séparation de l'énantiomère (-) du STB 6 a été réalisée par recristallisations successives dans le méthanol du sel formé à partir du STB 6 racémique avec l'acide (-) diparatoluoyl-L-tartrique.

Après purification du sel(-)(-), le composé (-) STB 6 base est obtenu par déplacement de sa combinaison saline par l'ammoniaque dilué et extraction au chloroforme.

**Isomère (+) STB 6**

L'énantiomère (+) du STB 6 a été séparé de la même manière que son inverse optique mais en utilisant de l'acide (+) diparatoluoyl-D-tartrique comme réactif de dédoublement. Le mélange racémique est extrait, après alcalinisation des filtrats des recristallisations précédentes et est enrichi en isomère (+).

**Exemple 2:**

**Préparation des isomères optiques purs de la phénacétyloxy-3 tétrahydro-5,6,13,13a 8H-dibenzo [a,g] quinolizine ou phénacétyloxy-3 berbine STB 69.**

**Isomère (-) et (+) STB 69**

Les isomères (+) et (-) du composé 69 ont été séparés de la même manière que les isomères (+) et (-) du composé STB 6 en recristallisant dans l'acétate d'éthyle.

**Exemple 3**

**Préparation des isomères optiques purs de la hydroxy-3 tétrahydro-5,6,13,13a 8H-dibenzo [a,g] quinolizine ou hydroxy-3 berbine.**

**Isomère (-) et (+) STB 4**

Les isomères (+) et (-) du composé STB 4 sont obtenus par hydrolyse en milieu acétochlorhydrique des isomères optiques purs du composé STB 69 préparés précédemment.

**Exemple 4**

**Préparation des isomères optiques purs de la diméthoxy-10,11 phénacétyloxy-3 tétrahydro-5,6,13,13a 8H-dibenzo [a,g] quinolizine ou diméthoxy-10,11 phénacétyloxy-3 berbine (STB 52).**

On a obtenu les isomères (+) et (-) de la diméthoxy- 10,11 phénacétyloxy-3 tétrahydro-5,6,13,13a 8H-dibenzo [a,g] quinolizine ou diméthoxy-10,11 phénacétyloxy-3 berbine STB 52 par estérification des composés STB 6 (+) et (-) respectivement.

Selon les modes opératoires décrits ci-dessus, on a obtenu les quantités de produits optiquement purs suivantes:

-11,5 g de STB 6 isomère (-) } à partir de 50 g de racémique base
-10,2 g de STB 6 isomère (+)
-6,0 g de STB 52 isomère (-)

-4,0 g de STB 52 isomère (+)
-7,2 g de STB 69 isomère (-) } à partir de 35 g de racémique base
-4,6 g de STB 69 isomère (+)
-3,4 g de STB 4 isomère (-)
-1,9 g de STB 4 isomère (+)

Dans le tableau ci-après, on a indiqué les valeurs des angles de rotation spécifique $[\alpha]^{20}_D$ des différents composés optiquement actifs isolés, et leurs principales caractéristiques physico-chimiques:

| Dérivés | Pouvoirs rotatoires spécifiques $[\alpha]^{20}_D$ (+) | (-) | Concentration g/100 ml | Solvant | F°C |
|---|---|---|---|---|---|
| STB 6, Base | + 298° | -297° | | +$CHCl_3$ | 224° |
| | | | | -$CHCl_3$ | 226° |
| STB 6, HCl | +202° | -202° | | +EtOH absolu dép | >200° |
| | | | | -EtOH absolu dép | >200° |
| STB 52 base | +230° | -232° | 0,2 | +$CHCl_2$ | 135° |
| | | | | -$CHCl_3$ | 136° |
| STB 52, HCl | +160° | -159° | | +EtOH absolu | 206° |
| | | | | -EtOH absolu | 205° |
| STB 69, base | +250° | -249° | | +$CHCl_3$ | 115° |
| | | | | -$CHCl_3$ | 114° |
| STB 69, HCl | +198° | -199° | | +EtOH absolu | 200° |
| | | | | -EtOH absolu | 198° |
| STB 4, base | +290° | -292° | | +$CHCl_3$ | 120-22° |
| | | | | -$CHCl_3$ | 120-22° |
| STB 4, HCl | +236° | -239° | | +EtOH 95° | dép <200° |
| | | | | -EtOH 95° | dép <200° |

dep=décomposition.

**Essais pharmacologiques**

**1) Effets hypotenseurs:**

L'étude a été réalisée chez le rat normotendu anesthésié au pentothal (40 $mg.kg^{-1}$ i.p.). La pression artérielle a été mesurée à l'aide d'un cathéter placé dans l'artère carotide et relié à une cellule de pression (Statham p 23 DB) et à un enregistreur selon la méthode décrite par Waeldele et Stoclet (J. Pharmacol. Paris 1973, 66, 357 - 366). Les dérivés sont administrés en doses croissantes toutes les 15 minutes par injection i.v.

On a représenté sur la figure 1 les effets sur la pression artérielle de doses croissantes des dérivés (+) et (-) STB 6 et (+) et (-) STB 4,15 minutes après administration i.v. au rat normotendu anesthésié. Chaque point est la moyenne des valeurs obtenues chez n animaux ± erreur type.

Sur cette figure 1, on a porté en abscisses les quantités de composé à tester administré, et, en ordonnées, la pression artérielle moyenne Δ P mm Hg.

La figure Ia est relative au composé STB 6

La figure Ib est relative au composé STB 4

Sur chacune des figures Ia, Ib, les courbes o——o sont relatives aux isomères (+), les courbes matérialisées par les •——• sont relatives aux isomères (-) et les courbes *——* sont relatives aux placebos.

Les résultats portés sur la figure 1 montrent que les isomères (-) du STB 6 et STB 4 produisent une chute significative de pression artérielle. Le (-) STB 4 et surtout le (-) STB 6 sont actifs à des doses faibles (respectivement 1 $mg.kg^{-1}$ et 0,5 $mg.kg^{-1}$).

Dans les mêmes conditions expérimentales, les dérivés (+) STB 6 et (+) STB 4 n'induisent pas de diminuion significative de la pression artérielle par rapport aux témoins.

**Recherche d'effets α-bloqueurs: étude de l'antagonisme entre les dérivés de la berbine et la phényléphrine sur l'aorte isolée de rat.**

L'évaluation quantitative de l'antagonisme entre les dérivés étudiés et la phényléphrine a été faite par l'analyse des relations effets-concentrations en présence et en absence d'antagoniste et le calcul du PA 2 à l'aide de la relation de Schild (Arunlasksha na O. et Schild H.P., Br. J. Pharmacol., 1959, 14, 48 - 58). L'analyse des relations effets-concentrations en réponse à la phényléphrine montre que l'antagonisme entre le (-) STB 6 et la phényléphrine est de nature

compétitive comme le montrent les résultats portés sur la figure II. Sur la figure IIa on a indiqué en ordonnées l'effet E (% de la contraction maximale) en l'absence et en présence de STB 6 (-), et en abscisses le Log de la concentration en phényléphrine; les différentes courbes sont relatives aux concentrations suivantes:

courbe •———• (-) STB 6 $5.10^{-8}$ M
courbe o———o (-) STB 6 $10^{-7}$M
courbe □———□ (-) STB 6 2.5 $10^{-7}$M
courbe Δ———Δ (-) STB 6 $5.10^{-7}$M
courbe ▲———▲ (-) STB 6 $10^{-6}$ M
courbe ■———■ (-) STB 6 $10^{-5}$ M
courbe *———* sans STB 6.

Les résultats sont la moyenne de 6 expériences (± sm).

La figure IIb est la représentation de Schild (x étant le rapport de concentration de phényléphrine qui donne un effet 50 % en présence et en l'absence du STB 6 (-). En ordonnées, on a porté Log (x - 1) et en abscisses Log [(-) STB 6].

Les paramètres de la relation de Schild ont également été mesurés en présence de STB 52 (-) dans les mêmes conditions expérimentales que pour STB 6(-).

Ces paramètres sont donnés ci-après. Paramètres de la relation de Schild observée en présence de (-) STB 52 et (-) STB 6

| | PA2 | n | r |
|---|---|---|---|
| (-) STB 52 | 7,0 | 1,00 | 0,99 |
| (-) STB 6 | 7,4 | 0,93 | 0,99 |

n = pente de la droite de Schild
r = coefficient de corrélation de la droite.

Les résultats ci-dessus montrent que (-) STB 52 est un antagoniste compétitif de la phényléphrine au même titre que le (-) STB 6.

Les dérivés (-) de STB 6 et STB 52 présentent une forte affinité pour le récepteur adrénergique. Par contre le dérivé (+) STB 6 présente un antagonisme de nature non compétitive vis-à-vis de la phényléphrine (figure III). La figure III représente l'antagonisme entre le (+) STB 6 et la phényléphrine sur l'aorte isolée de rat; sur cette figure, on a porté en ordonnées l'effet E (% de la contraction maximale) en l'absence et en présence de STB 6(+) et en abscisses le Log de la concentration en phényléphrine.

Les différentes courbes sont représentatives des concentrations ci-après:

*———* sans STB (+)
•———• STB 6 (+) $5.10^{-6}$ M
o———o STB 6 (+) $1.10^{-5}$ M
■———■ STB 6 (+) $5.10^{-5}$M
▲———▲ STB 6 (+) $1.10^{-4}$M

Les résultats représentés sur cette figure sont les moyennes ± sm de quatre expériences.

On indiquera de plus que les composés racémiques STB se comportent comme de puissants antagonistes non compétitifs de la phényléphrine. A cet effet, on pourra se référer à la page 40 de la description de la demande de brevet FR-A-2 469 413.

**3) Etude de l'inhibition de la stimulation sympathique in vivo**

Cette étude a été faite sur le dérivé le plus hypotenseur, le STB 6. Nous avons mesuré la réactivité vasculaire du territoire mésentérique perfusé in situ chez le rat (suivant Jackson et Campbell, Eur. J. Pharmacol., 1980, 66, 217 - 224). Après anesthésie du rat au pentothal (40 mg. $kg^{-1}$ i.p.) le territoire mésentérique a été perfusé à débit constant à partir d'un cathéter dérivant la circulation sanguine de l'aorte abdominale. On a mesuré l'effet vasoconstricteur de la stimulation électrique périartérielle des nerfs sympathiques (7 Hz) et de l'injection de noradrénaline (200 ng) dans le cathéter. Les effets des dérivés de la berbine administrés par voie i.v. ont été étudiés sur les deux types de réponses pressives. Les résultats obtenus sont portés sur les figures IVa et IVb sur lesquelles sont portées en ordonnées la pression artérielle moyenne Δ P mmHg et en abscisses le temps en minutes, l'injection du produit à tester ou du placébo (constitué par le même volume de sérum physiologique) étant effectuée au temps0.

La figure IVa est relative à la vasoconstriction provoquée par la stimulation électrique et la figure IVb à celle provoquée par la Noradrénaline.

Les courbes •——• correspondent au STB 6(+)
Les courbes o——o correspondent au STB 6(-).
Les courbes ■——■ correspondent au placébo.

Les résultats sont la moyenne ± sm de 5 expériences. On observe sur la figure IV que le dérivé (-) STB 6 administré à la dose de 0,5 $mg.kg^{-1}$ i.v., diminue la vasoconstriction du territoire mésentérique provoqué par stimulation électrique des nerfs sympathiques, sans modifier la réponse pressive à l'injection de noradrénaline. Administré dans les mêmes conditions et à la même dose, le dérivé (+) STB 6 n'a pas d'effet significatif. Ces résultats montrent que le (-) STB 6 inhibe in vivo soit la libération des médiateurs endogènes libérés par stimulation nerveuse soit la réponse pressive à ces médiateurs sans affecter la vasoconstriction induite par la noradrénaline exogène. Le (+) STB 6 ne présente pas cet effet dans les conditions expérimentales.

Les essais ci-dessus montrent que les dérivés (-) de la berbine possèdent des effets hypotenseurs et inhibent compétitivement l'effet vasoconstricteur de la phényléphrine (effet α-bloquant). Ces effets sont tous deux stéréospécifiques. En ce qui concerne le plus hypotenseur des dérivés étudiés, le STB 6, l'isomère (-) déprime la réponse pressive à la stimulation sympathique tandis que l'isomère (+) est dépourvu de cette propriété aux mêmes doses. L'isomère (+) se comporte comme un antagoniste non compétitif de la phényléphrine sur l'aorte isolée de rat.

**4) Etude de la sélectivité de la fixation des énantiomères de la berbine et de ses dérivés aux récepteurs $\alpha_1$ et $\alpha_2$ adrénergiques.**

Les expériences ont été réalisées sur un homogènat de cortex cérébral de rat, selon la méthode décrite par P. Greengross et R. Bremmerclaus Eur. J. Pharmacol. (1979) 55 323 - 326.

Les ligands utilisés sont respectivement le $^3$H-prazosin pour les sites $\alpha_1$ (KD 0,138 ± 0,02 n $mole.1^{-1}$ liaison maximale 200 ± 21 f moles. mg $protéines^{-1}$) et la $^3$H yohimbine pour les sites $\alpha_2$ ($K_D$ = 10,2 n $mole.1^{-1}$, liaison maximale 172,5 + 40,9 f moles.mg $protéines^{-1}$). Les résultats représentent la moyenne de 3 expériences. Les valeurs du Ki sont calculées d'après Cheng et Prussoff (Biochem. Pharmacol. 1973 22 3099 - 3108).

**Tableau I**

| | Liaison spécifique, Ki,n mole'$1^{-1}$ $\alpha_1$ | $\alpha_2$ |
|---|---|---|
| Prazosin | : 0.15 ± 0.03 | 382 ± 66 |
| Yohimbine | : 322 ± 26 | 13,7 ± 3,6 |
| berbine (-) | : 212 ± 26 | 140 ± 44 |
| (-)STB 6 | : 419 ± 132 | 1015 ± 335 |
| (-) STB 4 | : 462 ± 10 | 307 ± 118 |
| berbine (+) | :4730 ± 538 | 569 ± 137 |
| (+) STB 6 | :2931 ± 363 | 901 ± 299 |
| (+) STB 4 | :1220 ± 175 | 321 ± 72 |

. On constate que la berbine, isomère dextrogyre ou lévogyre a une affinité comparable pour les récepteurs $\alpha_1$, et $\alpha_2$, alors que l'isomère lévogyre du diméthoxy-10,11 hydroxy-3 tétrahydro-5,6,13,13a, 8H-dibenzo [a,g] quinolizine a une affinité marquée pour le récepteur $\alpha_1$, par rapport à l'$\alpha_2$.

**Toxicité:**

La $DL_{50}$ a été déterminée chez la souris. Les dérivés étudiés ont été administrés en solution aqueuse additionnée de 7 % de diméthylformamide, par gavage oesophagien de 0,4 ml par 209 de poids corporel, à des souris mâles pesant de 20 à 25 g. Dans ces conditions la $DL_{50}$ du (-) STB 6 est comprise entre 200 et 300 $mg.kg^{-1}$ celle du (-) STB 52 est supérieure à 800 $mg.kg^{-1}$ (30 % de mortalité à cette dose, limite de solubilité). Les dérivés (+) correspondants ne provoquent aucune mortalité à la dose de 800 $mg.kg^{-1}$ (limite de solubilité).

Les composés selon l'invention présentent donc des propriétés pharmacologiques qui permettent leur application dans le domaine cardio-vasculaire. Les doses à utiliser dépendent évidemment de l'état du patient et de l'affection cardio-vasculaire à traiter. Toutefois, on indiquera que des doses de 0,2 à 0,3 g par jour sont appropriées.

**Revendications** pour les Etats Contractants: BE, CH, LI, DE, GB, IT, LU, NL, SE

1. Enantiomères lévogyres de dérivés de la tétrahydro-5,6,13,13a 8H-dibenzo [a-g] quinolizine et de leurs sels pharmaceutiquement acceptables, qui répondent à la formule générale III:

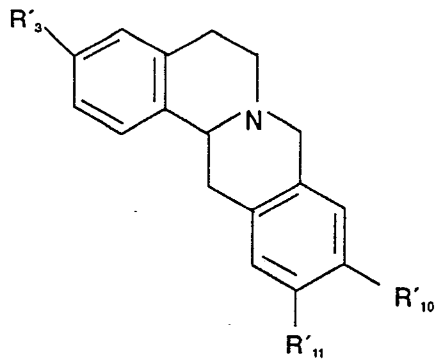

III

dans laquelle :

- R'3 est l'hydrogène, un groupe alkyle ou aryle ou un radical -OR- ou -SR, R étant l'hydrogène, un groupe alkyle ou aryle ou un groupe de formule $R''-\underset{\underset{O}{\|}}{C}-$, R" étant un groupe alkyle ou aryle,
- $R_{10}$ et $R'_{11}$, identiques ou différents, représentent l'hydrogène, un groupe hydroxy, alcoxy ou aryloxy ou $R'_{10}$ et $R'_{11}$ forment ensemble un groupe $-O-(CH_2)_n-O-$ dans lequel n vaut 1 à 3,

. à la condition que $R'_3$, $R'_{10}$ et $R'_{11}$ ne soient pas simultanément l'hydrogène; et,
. à la condition que lorsque $R'_{10}$ et $R'_{11}$ sont l'hydrogène, $R'_3$ ne soit pas un groupe alcoxy; et,
. à la condition que lorsque $R'_3$ est l'hydrogène, $R'_{10}$ et $R'_{11}$ ne soient pas un groupe hydroxy ou méthoxy;

les groupes alkyle et alcoxy ayant 1 à 12 atomes de carbone, et les groupes aryle étant choisis parmi les radicaux phényle ou phénylalkyle, où le groupe alkyle renferme de 1 à 4 atomes de carbone, et les hétérocycles aromatiques ayant 4 à 7 atomes de carbone et 1 à 4 hétéroatomes pouvant être l'oxygène, l'azote ou le soufre.

2. Enantiomère lévogyre d'hydroxy-3 tétrahydro-5,6,13,13a 8H-dibenzo [a-g] quinolizine, et ses sels pharmaceutiquement acceptables.

3. Enantiomère lévogyre de diméthoxy-10,11 hydroxy-3 tétrahydro-5,6,13,13a 8H-dibenzo [a-g] quinolizine et de ses sels pharmaceutiquement acceptables.

4. Esters des composés selon la revendication 3, tels que $R'_3$ de la formule III représente un groupe benzyle.

5. Procédé de préparation d'un énantiomère selon l'une quelconque des revendications 1 à 4, caractérisé en ce qu'il consiste à recristalliser le sel de l'acide (-) diparatoluoyl-2-tartrique du racémique de formule III jusqu'à l'obtention du sel du seul énantiomère lévogyre de formule III pur et à relibérer l'amine de son sel.

6. Procédé selon la revendication 5, caractérisé en ce que le solvant de recristallisation est choisi parmi l'éthanol, le méthanol, le chloroforme, l'acétate d'éthyle.

7. Procédé pour l'obtention des énantiomères lévogyres selon la revendication 1, dans lesquels $R'_3$ est un groupe $R''-\underset{\underset{O}{\|}}{C}-O$, caractérisé en ce qu'il consiste à estérifier l'énantiomère lévogyre correspondant de formule III dans laquelle $R'_3$ est le groupe hydroxy par action d'un chlorure d'acide de formule $R''-\underset{\underset{O}{\|}}{C}-Cl$.

8. Procédé pour l'obtention des énantiomères lévogyres selon la revendication 1, dans lesquels R'$_3$ est le groupe hydroxy, caractérisé en ce qu'il consiste à hydrolyser un énantiomère lévogyre correspondant de formule III dans laquelle R'$_3$ est le groupe R"COO tel que défini dans la revendication 1.

9. Composition pharmaceutique ayant des propriétés alpha-bloquantes, caractérisé en ce qu'elle contient, à titre d'ingrédient actif, l'énantiomère lévogyre d'un composé répondant à la formule III dans laquelle:

- R'$_3$ est l'hydrogène, un groupe alkyle, aryle, un radical OR ou SR, où R est l'hydrogène, un groupe alkyle, aryle ou $R''-\underset{\underset{O}{\|}}{C}-$, avec R" représentant un groupe alkyle ou aryle,
- R'$_{10}$ et R'$_{11}$, identiques ou différents, représentent l'hydrogène, un groupe hydroxy, alcoxy, aryloxy ou forment ensemble un groupe $O-(CH_2)_nO$ dans lequel n vaut 1 à 3,
- . à la condition que R'$_3$, R'$_{10}$ et R'$_{11}$ ne soient pas simultanément l'hydrogène; et,
- . à la condition que si R'$_{10}$ et R'$_{11}$ sont l'hydrogène ou un groupe alcoxy inférieur, R'$_3$ ne soit pas un groupe alcoxy;

les groupes alkyle et alcoxy ayant 1 à 12 atomes de carbone, les groupes alcoxy inférieurs, de 1 à 4 atomes de carbone, et les groupes aryle étant choisis parmi les radicaux phényle ou phénylalkyle, où le groupe alkyle renferme de 1 à 4 atomes de carbone, et les hétérocycles aromatiques ayant 4 à 7 atomes de carbone et 1 à 4 hétéroatomes pouvant être l'oxygène, l'azote ou le soufre.

10. Composition pharmaceutique selon la revendication 9, sous forme de dose unitaire utilisable dans le traitement des troubles cardiovasculaires et de l'hypertention artérielle.

**Revendications** pour l'Etat Contractant: AT

1. Procédé de préparation des énantiomères lévogyres de dérivés de la tétrahydro-5,6,13,13a 8H-dibenzo [a-g] quinolizine et de leurs sels pharmaceutiquement acceptables, qui répondent à la formule générale III:

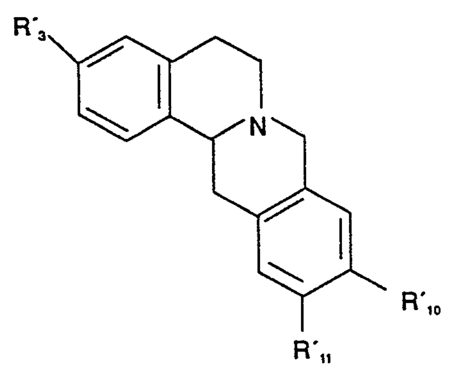

III

dans laquelle :

- R'$_3$ est l'hydrogène, un groupe alkyle ou aryle ou un radical -OR- ou -SR, R étant l'hydrogène, un groupe alkyle ou aryle ou un groupe de formule $R''-\underset{\underset{O}{\|}}{C}-$, R" étant un groupe alkyle ou aryle,
- R$_{10}$ et R'$_{11}$, identiques ou différents, représentent l'hydrogène, un groupe hydroxy, alcoxy ou aryloxy ou R'$_{10}$ et R'$_{11}$ forment ensemble un groupe $-O-(CH_2)_n-O-$ dans lequel n vaut 1 à 3,

- . à la condition que R'$_3$, R'$_{10}$ et R'$_{11}$ ne soient pas simultanément l'hydrogène; et,
- . à la condition que lorsque R'$_{10}$ et R'$_{11}$ sont l'hydrogène, R'$_3$ ne soit pas un groupe alcoxy; et,
- . à la condition que lorsque R'$_3$ est l'hydrogène, R'$_{10}$ et R'$_{11}$ ne soient pas un groupe hydroxy ou méthoxy;

les groupes alkyle et alcoxy ayant 1 à 12 atomes de carbone, et les groupes aryle étant choisis parmi les radicaux phényle ou phénylalkyle, où le groupe alkyle renferme de 1 à 4 atomes de carbone, et les hétérocycles aromatiques ayant 4 à 7 atomes de carbone et 1 à 4 hétéroatomes pouvant être l'oxygène, l'azote ou le soufre, qui consiste à recristalliser un sel du mélange racémique de la base de formule III avec un réactif acide de dédoublement, puis à libérer l'énantiomètre de son sel et à préparer un autre sel si nécessaire.

2. Procédé de préparation d'un énantiomère selon la revendication 1, caractérisé en ce qu'il consiste à recristalliser le sel de l'acide (-) diparatoluoyl-2-tartrique du racémique de formule III jusqu'à obtention du sel du seul énantiomètre lévogyre de formule III pur et à libérer l'amine de son sel.

3. Procédé selon la revendication 2, caractérisé en ce que le solvant de recristallisation est choisi parmi l'éthanol, le méthanol, le chloroforme, l'acétate d'éthyle.

4. Procédé pour l'obtention des énantiomères lévogyres définis à la revendication 1, dans lesquels $R'_3$ est un groupe R"–C–O, caractérisé en ce qu'il consiste à
||
O
estérifier l'énantiomère lévogyre correspondant de formule III dans laquelle $R'_3$ est le groupe hydroxy par action d'un chlorure d'acide de formule R"–C–Cl.
||
O

5. Procédé pour l'obtention des énantiomères lévogyres définis à la revendication 1, dans lesquels $R'_3$ est le groupe hydroxy, caractérisé en ce qu'il consiste à hydrolyser un énantiomère lévogyre correspondant de formule III dans laquelle $R'_3$ est le groupe R"COO tel que défini dans la revendication 1.

6. Procédé selon l'une des revendications 1 à 5 pour l'obtention de l'énantiomère lévogyre d'hydroxy-3 tétrahydro-5,6,13,13a 8H-dibenzo [a,g] quinolizine et de ses sels pharmaceutiquement acceptables.

7. Procédé selon l'une des revendications 1 à 5 pour l'obtention de l'énantiomère lévogyre de diméthoxy-10,11 hydroxy-3 tétrahydro-5,6,13,13a 8H-dibenzo [a,g] quinolizine et de ses sels pharmaceutiquement acceptables.

8. Procédé de préparation d'une composition pharmaceutique ayant des propriétés alpha-bloquantes, qui consiste à mettre sous forme de dose unitaire, un véhicule pharmaceutiquement acceptable et une quantité thérapeutiquement efficace d'énantiomère lévogyre d'un composé répondant à la formule III de la revendication 1 dans laquelle:

- $R'_3$ est l'hydrogène, un groupe alkyle, aryle, un radical OR ou SR où R est l'hydrogène, un groupe alkyle, aryle ou R"–C–, avec R" représentant un groupe alkyle ou aryle,
  ||
  O
- $R'_{10}$ et $R'_{11}$, identiques ou différents, représentent l'hydrogène, un groupe hydroxy, alcoxy, aryloxy ou forment ensemble un groupe $O\text{-}(CH_2)_nO$ dans lequel n vaut 1 à 3

. à la condition que $R'_3$, $R'_{10}$ et $R'_{11}$ ne soient pas simultanément l'hydrogène; et,
. à la condition que si $R'_{10}$ et $R'_{11}$ sont l'hydrogène ou un groupe alcoxy inférieur, $R'_3$ ne soit pas un groupe alcoxy;

les groupes alkyle et alcoxy ayant 1 à 12 atomes de carbone, les groupes alcoxy inférieurs, de 1 à 4 atomes de carbone, et les groupes aryle étant choisis parmi les radicaux phényle ou phénylalkyle, où le reste alkyle renferme de 1 à 4 atomes de carbone, et les hétérocycles aromatiques ayant 4 à 7 atomes de carbone et 1 à 4 hétéroatomes pouvant être l'oxygène, l'azote ou le soufre.

9. Procédé selon la revendication 8, dans lequel on met en oeuvre l'énantiomère lévogyre d'hydroxy-3 tétrahydro-5,6,13,13a 8H-dibenzo [a,g] quinolizine et ses sels pharmaceutiquement acceptables.

10. Procédé selon la revendication 8, dans lequel on met en oeuvre l'énantiomère lévogyre de diméthoxy-10,11 hydroxy-3 tétrahydro-5,6,13,13a 8H-dibenzo [a,g] quinolizine et de ses sels pharmaceutiquement acceptables.

**Claims** for the Contracting States BE, CH, LI, DE, GB, IT, LU, NL, SE

1. Laevorotatory enantiomers of derivatives of 5, 6,13, 13a-tetrahydro-8H-dibenzo[a-g]quinolizine and of their pharmaceutically acceptable salts, which correspond to the general formula III:

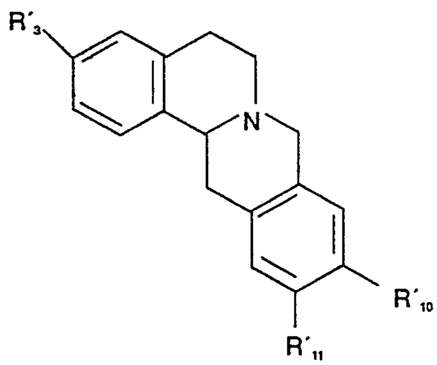

III

in which:

- R'3 is hydrogen, an alkyl or aryl group or a radical -OR- or -SR, R being hydrogen, an alkyl or aryl group or a group of formula $R''\text{-}\underset{\underset{O}{\|}}{C}\text{-}$, R" being an alkyl or aryl group,
- R'10 and R'11 which are identical or different, denote hydrogen, a hydroxyl, alkoxy or aryloxy group or R'10 and R'11 together form a group $-O-(CH_2)n-O-$ in which n has the value 1 to 3,

  on condition that R'3, R'10 and R'11 are not simultaneously hydrogen,; and
  on condition that, when R'10 and R'11 are hydrogen, R'3 is not an alkoxy group; and
  on condition that, when R'3 is hydrogen, R'10 and R'11 are not a hydroxyl or methoxy group;

the alkyl and alkoxy groups containing 1 to 12 carbon atoms, and the aryl groups being chosen from phenyl or phenylalkyl radicals, where the alkyl group contains from 1 to 4 carbon atoms, and the aromatic heterocyclic rings containing 4 to 7 carbon atoms and 1 to 4 heteroatoms which may be oxygen, nitrogen or sulphur.

2. Laevorotatory enantiomer of 3-hydroxy-5,6,13,13-atetrahydro-8H-dibenzo[a-g]quinolizine and its pharmaceutically acceptable salts.

3. Laevorotatory enantiomer of 10,11-dimethoxy-3-hydroxy-5,6,13,13a-tetrahydro-8H-dibenzo[a-g]quinolizine and of its pharmaceutically acceptable salts.

4. Esters of the compounds according to Claim 3, such that R'3 of formula III denotes a benzyl group.

5. Process for the preparation of an enantiomer according to any one of Claims 1 to 4, characterized in that it consists in recrystallizing the (-) 2-di-paratoluolyltartaric acid salt of the racemate of formula III until the salt of only the laevorotatory enantiomer of formula III is obtained pure and in reliberating the amine from its salt.

6. Process according to Claim 5, characterized in that the recrystallization solvent is chosen from ethanol, methanol, chloroform and ethyl acetate.

7. Process for obtaining the laevorotatory enantiomers according to Claim 1, in which R'3 is an $R''\text{-}\underset{\underset{O}{\|}}{C}\text{-}O$ group, characterized in that it consists in esterifying the corresponding laevorotatory enantiomer of formula III in which R'3 is the hydroxyl group by reaction with an acid chloride of formula $R''\text{-}\underset{\underset{O}{\|}}{C}\text{-}Cl$.

8. Process for obtaining the laevorotatory enantiomers according to Claim 1, in which $R'_3$ is the hydroxyl group, characterized in that it consists in hydrolysing a corresponding laevorotatory enantiomer of formula III in which $R'_3$ is the R"COO group such as defined in Claim 1.

9. Pharmaceutical composition having alpha-blocking properties, characterized in that it contains, as an active ingredient, the laevorotatory enantiomer of a compound corresponding to the formula III in which:

- $R'_3$ is hydrogen, an alkyl or aryl group, a radical OR or SR, where R is hydrogen, an alkyl, aryl or R"-C-, group, with R" denoting an alkyl or aryl group,
  ||
  O
- $R'_{10}$ and $R'_{11}$' which are identical or different, denote hydrogen or a hydroxyl, alkoxy or aryloxy group or together form a group $O-(CH_2)_nO$ in which n has the value 1 to 3,
  on condition that $R'_3$, $R'_{10}$ and $R'_{11}$ are not simultaneously hydrogen; and
  on condition that, if $R'_{10}$ and $R'_{11}$ are hydrogen or a lower alkoxy group, $R'_3$ is not an alkoxy group;

the alkyl and alkoxy groups containing 1 to 12 carbon atoms, the lower alkoxy groups from 1 to 4 carbon atoms, and the aryl groups being chosen from phenyl or phenylalkyl radicals, where the alkyl group contains from 1 to 4 carbon atoms, and the aromatic heterocyclic rings containing 4 to 7 carbon atoms and 1 to 4 heteroatoms which may be oxygen, nitrogen or sulphur.

10. Pharmaceutical composition according to Claim 9, in the form of a unit dose which can be employed in the treatment of cardiovascular disorders and of arterial hypertension.

**Claims** for the Contracting State: AT

1. Process for the preparation of the laevorotatory enantiomers of derivatives of 5, 6,13,13a-tetrahydro-8Hdibenzo[a-g]quinolizine and of their pharmaceutically acceptable salts, which correspond to the general formula III:

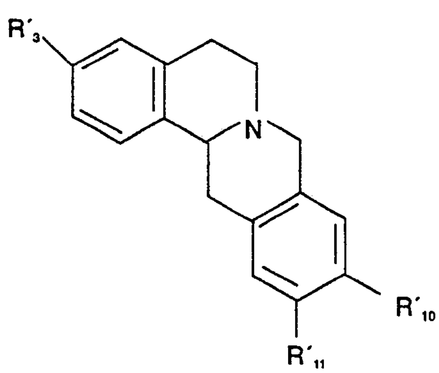

III

in which:

- R'3 is hydrogen, an alkyl or aryl group or a radical -OR or -SR, R being hydrogen, an alkyl or aryl group or a group of formula R"-C-, R" being an alkyl or aryl group,
  ||
  O
- $R'_{10}$ and $R'_{11}$, which are identical or different, denote hydrogen, a hydroxyl, alkoxy or aryloxy group or $R'_{10}$ and $R'_{11}$ together form a group $-O-(CH_2)_n-O-$ in which n has the value 1 to 3,
  on condition that $R'_3$, $R'_{10}$ and $R'_{11}$ are not simultaneously hydrogen,; and
  on condition that, when $R'_{10}$ and $R'_{11}$ are hydrogen, $R'_3$ is not an alkoxy group; and
  on condition that, when $R'_3$ is hydrogen, $R'_{10}$ and $R'_{11}$ are not a hydroxyl or methoxy group;

the alkyl and alkoxy groups containing 1 to 12 carbon atoms, and the aryl groups being chosen from phenyl or phenylalkyl radicals, where the alkyl group contains from 1 to 4 carbon atoms, and the aromatic heterocyclic rings containing 4 to 7 carbon atoms and 1 to 4 heteroatoms which may be oxygen, nitrogen or sulphur,

which consists in recrystallizing a salt of the racemic mixture of the base of formula III with an acidic resolving reactant, and in then liberating the enantiomer from its salt and in preparing another salt if necessary.

2. Process for the preparation of an enantiomer according to Claim 1, characterized in that it consists in recrystallizing the (-) 2-di-para-toluolyltartaric acid salt of the racemate of formula III until the salt of only the laevorotatory enantiomer of formula III is obtained pure and in liberating the amine from its salt.

3. Process according to Claim 2, characterized in that the recrystallization solvent is chosen from ethanol, methanol, chloroform and ethyl acetate.

4. Process for obtaining the laevorotatory enantiomers defined in Claim 1, in which $R'_3$ is a R"-C-O
||
O
group, characterized in that it consists in esterifying the corresponding laevorotatory enantiomer of formula III in which $R'_3$ is the hydroxyl group by reaction with an acid chloride of formula R"-C-Cl.
||
O

5. Process for obtaining the laevorotatory enantiomers defined in Claim 1, in which $R'_3$ is the hydroxyl group, characterized in that it consists in hydrolysing a corresponding laevorotatory enantiomer of formula III in which $R'_3$ is the R"COO group such as defined in Claim 1.

6. Process according to one of Claims 1 to 5 for obtaining the laevorotatory enantiomer of 3-hydroxy-5,6,13,13a-tetrahydro-8H-dibenzo[a,g]quinolizine and of its pharmaceutically acceptable salts.

7. Process according to one of Claims 1 to 5 for obtaining the laevorotatory enantiomer of 10,11-dimethoxy-3-hydroxy-5,6,13,13a-tetrahydro-8H-dibenzo[a,g]quinolizine and of its pharmaceutically acceptable salts.

8. Process for the preparation of a pharmaceutical composition having alpha-blocking properties, which consists in putting in the form of a unit dose a pharmaceutically acceptable carrier and a therapeutically effective quantity of laevorotatory enantiomer of a compound corresponding to the formula III of Claim 1, in which:

- $R'_3$ is hydrogen, an alkyl or aryl group, a radical OR or SR, where R is hydrogen, an alkyl, aryl or R"-C- group, with R" denoting an alkyl or aryl group,
  ||
  O
- $R'_{10}$ and $R'_{11}$' which are identical or different, denote hydrogen or a hydroxyl, alkoxy or aryloxy group or together form a group $O-(CH_2)_nO$ in which n has the value 1 to 3
  on condition that $R'_3$, $R'_{10}$ and $R'_{11}$ are not simultaneously hydrogen; and
  on condition that, if $R'_{10}$ and $R'_{11}$ are hydrogen or a lower alkoxy group, $R'_3$ is not an alkoxy group;

the alkyl and alkoxy groups containing 1 to 12 carbon atoms, the lower alkoxy groups from 1 to 4 carbon atoms, and the aryl groups being chosen from phenyl or phenylalkyl radicals, where the alkyl residue contains from 1 to 4 carbon atoms, and the aromatic heterocyclic rings containing 4 to 7 carbon atoms and 1 to 4 heteroatoms which may be oxygen, nitrogen or sulphur.

9. Process according to Claim 8, in which there is employed the laevorotatory enantiomer of 3-hydroxy-5,6,13,13a-tetrahydro-8H-dibenzo[a,g]quinolizine and its pharmaceutically acceptable salts.

10. Process according to Claim 8, in which there is employed the laevorotatory enantiomer of 10,11-dimethoxy-3-hydroxy-5,6,13,13a-tetrahydro-8H-dibenzo[a,g]quinolizine and of its pharmaceutically acceptable salts.

**Patentansprüche** für die Vertragsstaaten BE, CH, LI, DE, GB, IT, LU, NL, SE

1. Linksdrehende Enantiomere von Derivaten des 5,6,13,13aTetrahydro-8H-dibenzo[a-g]-chinolizin und ihre pharmazeutisch akzeptablen Salze der allgemeinen Formel III:

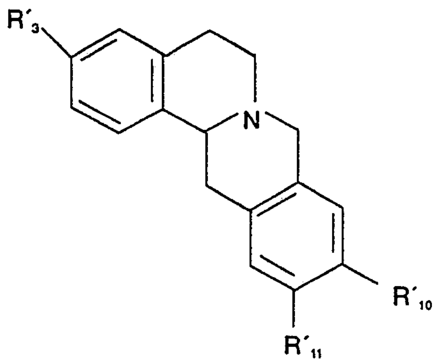

III

worin

R'3 Wasserstoff, eine Alkyl- oder Arylgruppe oder ein -OR- oder -SR-Rest ist, worin R Wasserstoff, eine Alkyl- oder Arylgruppe oder eine Gruppe der Formel R"-C- ist und R" eine Alkyl- oder Arylgruppe ist,
||
O

R'10 und R'11 gleich oder verschieden sind und Wasserstoff, eine Hydroxy-, Alkoxy- oder Aryloxygruppe darstellen oder zusammen eine Gruppe $-O-(CH_2)_n-O-$ bilden, in der n 1 bis 3 beträgt,

- mit der Maßgabe, daß R'3, R'10 und R'11 nicht gleichzeitig Wasserstoff sind; und
- mit der Maßgabe, daß, wenn R'10 und R'11 Wasserstoff sind, R'3 keine Alkoxygruppe ist; und
- mit der Maßgabe, daß, wenn R'3 Wasserstoff ist, R'10 und R'11 keine Hydroxy- oder Methoxygruppe darstellen;

wobei die Alkyl- und Alkoxygruppen 1 bis 12 C-Atome haben und die Arylgruppen aus der Gruppe der Phenyl- oder Phenylalkylreste ausgewählt sind, worin die Alkylgruppe 1 bis 4 C-Atome enthält und die aromatischen Heterozyklen 4 bis 7 C-Atome und 1 bis 4 Heteroatome, die Sauerstoff, Stickstoff oder Schwefel sein können, aufweisen.

2. Linksdrehendes Enantiomer von 3-Hydroxy-5,6,13,13a-tetrahydro-8H-dibenzo[a-g]-chinolizin und seine pharmazeutisch akzeptablen Salze.

3. Linksdrehendes Enantiomer von 10,11-Dimethoxy-3-hydroxy-5,6,13,13a-tetrahydro-8H-dibenzo[a-g]-chinolizin und seine pharmazeutisch akzeptablen Salze.

4. Ester der Verbindungen nach Anspruch 3, dadurch gekennzeichnet, daß R'3 der Formel III eine Benzylgruppe darstellt.

5. Verfahren zur Herstellung eines Enantiomeren nach irgendeinem der Ansprüche 1 bis 4, dadurch gekennzeichnet, daß man das Salz der (-)2-Diparatoluoylweinsäure des racemischen Gemischs der Formel III umkristallisiert, bis man das Salz des einzigen linksdrehenden Enantiomers der Formel III rein erhält, und das Amin von seinem Salz freisetzt.

6. Verfahren nach Anspruch 5, dadurch gekennzeichnet, daß das Lösungsmittel für die Umkristallisation aus Ethanol, Methanol, Chloroform und Ethylacetat ausgewählt ist.

7. Verfahren zur Herstellung linksdrehender Enantiomere nach Anspruch 1, worin R'3 eine Gruppe R"-C-O ist,
||
O

dadurch gekennzeichnet, daß man das linksdrehende Enantiomer nach der Formel III, worin R'3 die Hydroxygruppe ist, mit Hilfe eines Säurechlorids der Formel R"-C-Cl verestert.
||
O

8. Verfahren zur Herstellung der linksdrehenden Enantiomeren nach Anspruch 1, in denen R'3 die Hydroxygruppe ist, dadurch gekennzeichnet, daß man ein linksdrehendes Enantiomer der Formel III, in der R'3 die Gruppe R"-COO, wie sie im Anspruch 1 definiert ist, darstellt, hydrolysiert.

9. Pharmazeutische Zusammensetzung mit alpha-blockierenden Eigenschaften, dadurch gekennzeichnet, daß sie als Wirkstoff das linksdrehende Enantiomer einer Verbindung nach Formel III enthält, worin:

R'3 Wasserstoff, eine Alkyl- oder Arylgruppe, ein OR-Rest oder ein SR-Rest ist, worin R Wasserstoff, eine Alkyl- oder Arylgruppe oder R"-C- ist, und R" eine Alkyl- oder Arylgruppe darstellt,

||
O

$R'_{10}$ und $R'_{11}$ gleich oder verschieden sind und Wasserstoff, eine Hydroxy-, Alkoxy-, Aryloxygruppe darstellen oder zusammen eine Gruppe $O\text{-}(CH_2)_nO$, in der n 1 bis 3 beträgt, bilden,

- mit der Maßgabe, daß $R'_3$, $R'_{10}$ und $R'_{11}$ nicht gleichzeitig Wasserstoff sind; und
- mit der Maßgabe, daß, wenn R'10 und $R'_{11}$ Wasserstoff oder eine Niederalkoxygruppe sind, $R'_3$ keine Alkoxygruppe ist;

wobei die Alkyl- und Alkoxygruppen 1 bis 12 C-Atome haben, die Niederalkoxygruppen 1 bis 4 C-Atome besitzen und die Arylgruppen aus der Gruppe der Phenyl- oder Phenylalkylreste ausgewählt sind, worin die Alkylgruppe 1 bis 4 C-Atome enthält und die aromatischen Heterocyclen 4 bis 7 C-Atome und 1 bis 4 Heteroatome aufweisen, die Sauerstoff, Stickstoff oder Schwefel sein können.

10. Pharmazeutische Zusammensetzung nach Anspruch 9, in Form einer Einheitsdosis, verwendbar in der Behandlung von kardiovaskulären Störungen und von arteriellem Bluthochdruck.

**Patentansprüche** für den Vertragsstaat: AT

1. Verfahren zur Herstellung von linksdrehenden Enantiomeren von Derivaten des 5,6,13,13a-Tetrahydro-8H-dibenzo[a-g]-chinolizin und ihrer pharmazeutisch akzeptablen Salze der allgemeinen Formel III:

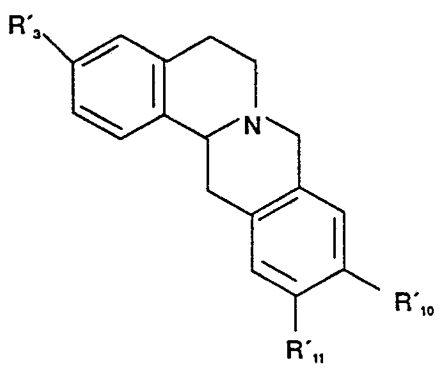

III

worin

R'3 Wasserstoff, eine Alkyl- oder Arylgruppe oder ein -OR- oder -SR-Rest ist, worin R Wasserstoff, eine Alkyl- oder Arylgruppe oder eine Gruppe der Formel R"-C- ist und R" eine Alkyl- oder Arylgruppe ist,

||
O

R'10 und $R'_{11}$ gleich oder verschieden sind und Wasserstoff, eine Hydroxy-, Alkoxy- oder Aryloxygruppe darstellen oder zusammen eine Gruppe $-O-(CH_2)_n-O-$ bilden, in der n 1 bis 3 beträgt,

- mit der Maßgabe, daß $R'_3$, $R'_{10}$ und $R'_{11}$ nicht gleichzeitig Wasserstoff sind; und
- mit der Maßgabe, daß, wenn $R'_{10}$ und $R'_{11}$ Wasserstoff sind, $R'_3$ keine Alkoxygruppe ist; und
- mit der Maßgabe, daß, wenn $R'_3$ Wasserstoff ist, $R'_{10}$ und $R'_{11}$ keine Hydroxy- oder Methoxygruppe darstellen;

wobei die Alkyl- und Alkoxygruppen 1 bis 12 C-Atome haben und die Arylgruppen aus der Gruppe der Phenyl- oder Phenylalkylreste ausgewählt sind, worin die Alkylgruppe 1 bis 4 C-Atome enthält und die aromatischen Heterozyklen 4 bis 7 C-Atome und 1 bis 4 Heteroatome, die Sauerstoff, Stickstoff oder Schwefel sein können, aufweisen, das darin besteht, daß man ein Salz eines racemischen Gemischs der Base der Formel III mit einem sauren Spaltungsreagens umkristallisiert, dann das Enantiomere aus seinem Salz freisetzt und ein anderes Salz, wenn nötig, herstellt.

2. Verfahren zur Herstellung eines Enantiomeren nach Anspruch 1, dadurch gekennzeichnet, daß man das Salz der (-)2-Diparatoluoylweinsäure des racemischen Gemischs der Formel III umkristallisiert, bis man das Salz des einzigen linksdrehenden Enantiomeren der Formel III rein erhält, und das Amin von seinem Salz freisetzt.

3. Verfahren nach Anspruch 2, dadurch gekennzeichnet, daß das Lösungsmittel für die Umkristallisation aus Ethanol, Methanol, Chloroform und Ethylacetat ausgewählt ist.

4. Verfahren zur Herstellung linksdrehender Enantiomeren nach Anspruch 1, worin R'3 eine Gruppe R"-C-O ist,
||
O
dadurch gekennzeichnet, daß man das linksdrehende Enantiomer nach der Formel III, worin $R'_3$ die Hydroxygruppe ist, mit Hilfe eines Säurechlorids der Formel R"-C-Cl verestert.
||
O

5. Verfahren zur Herstellung der linksdrehenden Enantiomeren nach Anspruch 1, in denen $R'_3$ die Hydroxygruppe ist, dadurch gekennzeichnet, daß man ein linksdrehendes Enantiomer der Formel III, in der $R'_3$ die Gruppe R"-COO, wie sie im Anspruch 1 definiert ist, darstellt, hydrolysiert.

6. Verfahren nach einem der Ansprüche 1 bis 5 zur Herstellung des linksdrehenden Enantiomeren von 3-Hydroxy-5,6,13,13a-tetrahydro-8H-dibenzo-[a-g]-chinolizin und seiner pharmazeutisch akzeptablen Salze.

7. Verfahren nach einem der Ansprüche 1 bis 5 zur Herstellung des linksdrehenden Enantiomeren von 10,11-Dimethoxy-3-hydroxy-5,6,13,13a-tetrahydro-8H-dibenzo-[a-g]-chinolizin und seiner pharmazeutisch akzeptablen Salze.

8. Verfahren zur Herstellung einer pharmazeutischen Zusammensetzung mit alpha-blockierenden Eigenschaften, das darin besteht, daß man einen brauchbaren pharmazeutischen Träger und eine therapeutisch effektive Menge eines linksdrehenden Enantiomeren einer Verbindung nach Formel III des Anspruchs 1 in die Form einer Einheitsdosis bringt, wobei in der Formel III die Reste folgendermaßen definiert sind:

R'3 ist Wasserstoff, eine Alkyl- oder Arylgruppe, ein OR-Rest oder ein SR-Rest, worin R Wasserstoff, eine Alkyl- oder Arylgruppe oder R"-C- ist, und R" eine Alkyl- oder Arylgruppe ist,
||
O

R'10 und $R'_{11}$ sind gleich oder verschieden und stellen Wasserstoff, eine Hydroxy-, Alkoxy-, oder Aryloxygruppe dar oder bilden zusammen eine Gruppe $O-(CH_2)_nO$, in der n 1 bis 3 beträgt,

- mit der Maßgabe, daß $R'_3$, $R'_{10}$ und $R'_{11}$ nicht gleichzeitig Wasserstoff sind; und
- mit der Maßgabe, daß, wenn $R'_{10}$ und $R'_{11}$ Wasserstoff oder eine Niederalkoxygruppe sind, $R'_3$ keine Alkoxygruppe ist;

wobei die Alkyl- und Alkoxygruppen 1 bis 12 C-Atome haben, die Niederalkoxygruppen 1 bis 4 C-Atome besitzen und die Arylgruppen aus der Gruppe der Phenyl- oder Phenylalkylreste ausgewählt sind, worin die Alkylgruppe 1 bis 4 C-Atome enthält und die aromatischen Heterocyclen 4 bis 7 C-Atome und 1 bis 4 Heteroatome aufweisen, die Sauerstoff, Stickstoff oder Schwefel sein können.

9. Verfahren nach Anspruch 8, in dem das linksdrehende Enantiomer 3-Hydroxy-5,6,13,13a-tetrahydro-8H-dibenzo-[a-g]-chinolizin und seine pharmazeutisch verträglichen Salze eingesetzt wird.

10. Verfahren nach Anspruch 8, in dem das linksdrehende Enantiomer 10,11-Dimethoxy-3-hydroxy-5,6,13,13a-tetrahydro-8H-dibenzo-[a-g]-chinolizin und seine pharmazeutisch verträglichen Salze eingesetzt wird.

FIG.1a

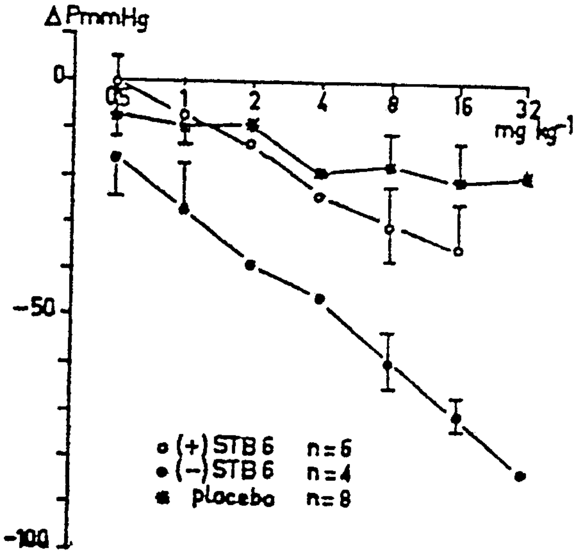
ΔPmmHg
0
-50
-100
0.5
1
2
4
8
16
32
mg kg-1
o (+) STB 6 n=6
● (-) STB 6 n=4
* placebo n=8

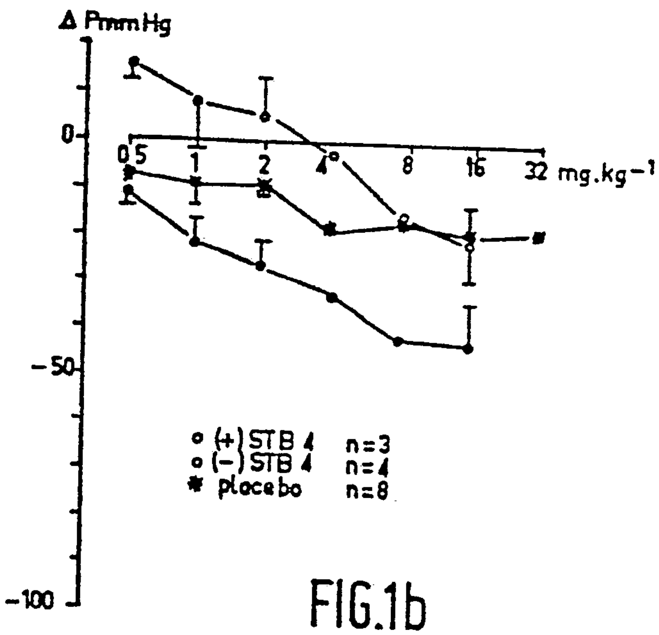
Δ PmmHg
0
-50
-100
0.5
1
2
4
8
16
32 mg.kg-1
o (+) STB 4 n=3
o (-) STB 4 n=4
* placebo n=8

FIG.1b

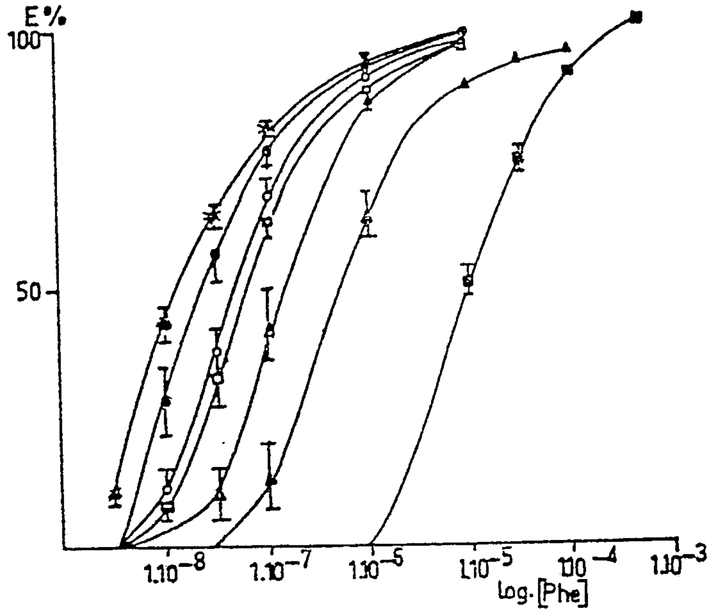
E%
100
50
1.10⁻⁸
1.10⁻⁷
1.10⁻⁶
1.10⁻⁵
1.10⁻⁴
1.10⁻³
log.[Phe]

FIG.2a

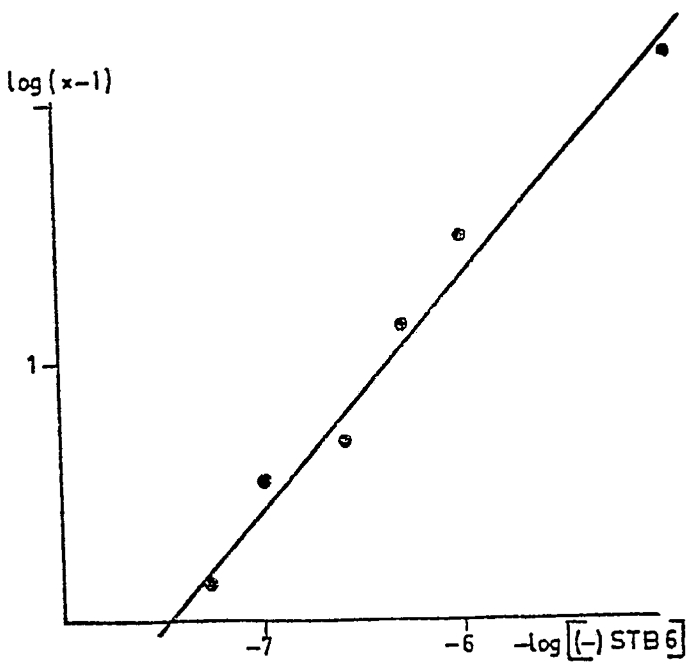
log(x−1)
1
−7
−6
−log[(−) STB 6]

FIG.2b

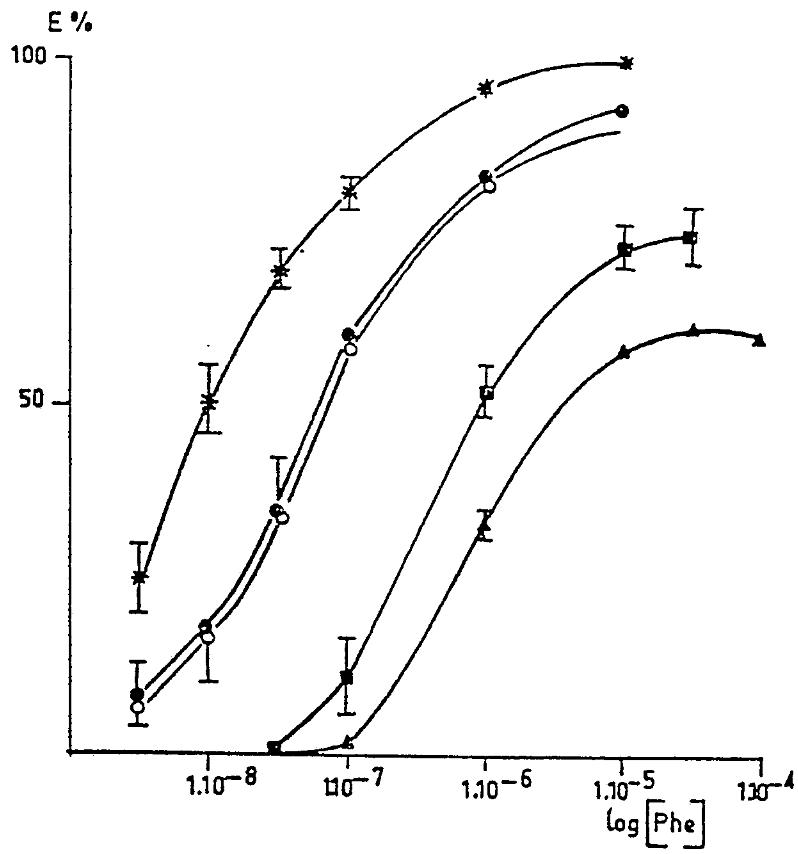


FIG.3

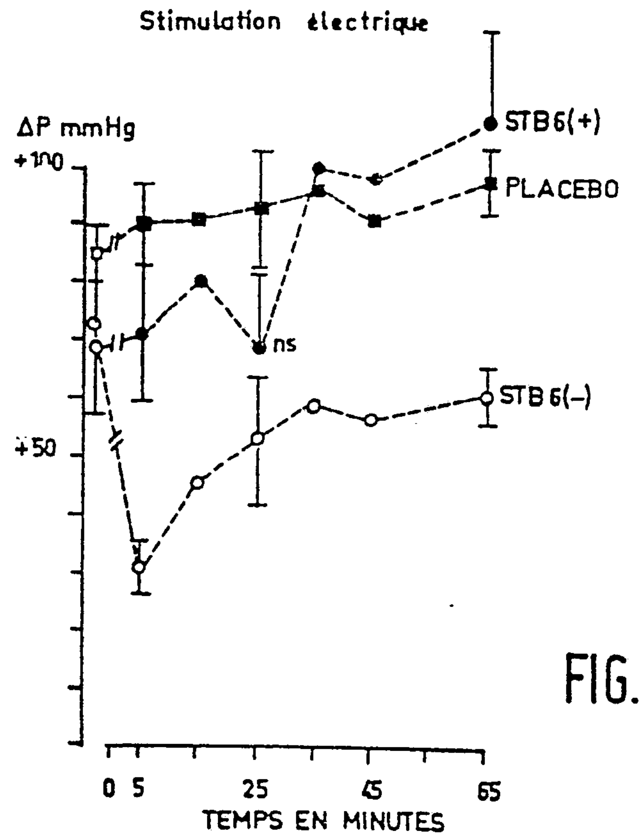
Stimulation électrique
ΔP mmHg
+100
+50
STB 6(+)
PLACEBO
ns
STB 6(−)
0 5
25
45
65
TEMPS EN MINUTES

FIG.4a

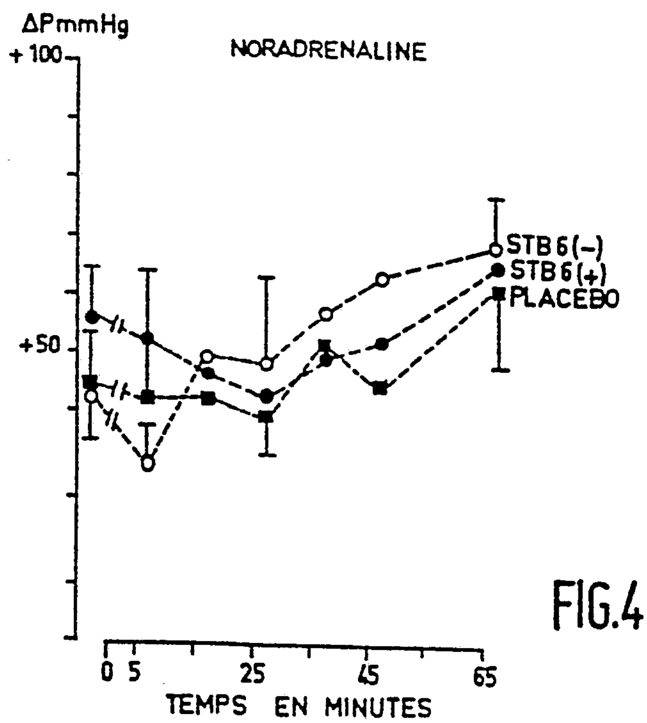
ΔPmmHg
+100
NORADRENALINE
+50
STB 6(−)
STB 6(+)
PLACEBO
0 5
25
45
65
TEMPS EN MINUTES

FIG.4b